# EUROPEAN PATENT APPLICATION

(11) **EP 4 599 817 A1**
(43) Date of publication of application: **13.08.2025**
(21) Application number: 24157012.6
(22) Date of filing: 12.02.2024

(54) **IMPACT ON ENAMEL AND DENTINE HYPERSENSITIVITY THROUGH MEDIATED IMPACT OF ANTAGONISTS ON TRPV1 AND TRCP5 CHANNELS IN ORAL CARE PRODUCTS**

(71) Applicant: SkyLab AG, 1066 Epalinges (CH)
(72) Inventor: Belous, Elena Yur'evna, 121309 MOSCOW (RU); Ivanova, Angelina Dmitrievna, London, NW61NE (GB)
(74) Representative: Glawe, Delfs, Moll

(57) **Abstract**

The invention relates to a composition comprising galangin, oleic acid and adenosine. Said composition can be used as an oral care composition or as a medicament for use in the prevention from or treatment of a disease or symptoms of a disease in a subject, e.g. dentin hypersensitivity, periodontitis and/or gingivitis.

## Description

### FIELD OF INVENTION

The present invention relates to a new invention which assists in solving the issue of dental enamel and dentine hypersensitivity via a new combination containing galangin, oleic acid and adenosine, in oral care products.

### STATE OF THE ART

Dentine hypersensitivity (DHS) is one of the most frequent complaints in dental patients; however there are no routine recommendations on differential diagnostics and selection of reliable therapeutic decision for this condition. Hypersensitivity occurs when dentine is exposed and gingival soft tissue is recessed. This can be occasioned by excessive or inadequate oral care, as well as by enamel erosion and pathological enamel attrition. Teeth with DHS become sensitive to normally safe external stimuli. A tender touch, a slight cold, hot or chemical exposure (sour or sweet fruit, food products, drinks) and air flows can cause short term pangs, which may impact daily routine, including eating, drinking, talking and toothbrushing. A more severe form of DHS can continue over 6 months and become a stationary stimulus causing psychological and emotional pressure [23, 31], which can induce the condition of chronic dental pain requiring special treatment. It is also known that the life quality of patients suffering from dentine hypersensitivity, connected with oral health can be improved after successful DHS treatment [15]. The share of population over 20 years old suffering from this condition makes 8 to 57% depending on place of residence and life style [18, 19, 24].

Dentine hypersensitivity mechanism is understudied; however, there exist several basic theories explaining it. Currently the following three basic theories are used to postulate this physiopathogenesis, namely: hydrodynamic theory, nerve theory and odontoblast theory.

The hydrodynamic theory of sensitivity is most widely used. The backbone of the theory is based on the fact that rapid liquid flow in dentine tubules causes sensory nerve activation in the dental pulp and internal tooth dentine area. Specific stimuli, such as temperature changes, chemical exposure and strong air flow cause pressure change in dentine, which is able to impulse specific dental nerves thus inducing sharp pain [38].

The odontoblast theory, or odontoblast conversion theory is based on the fact that odontoblasts act as receptor cells and transfer impulses to nerve terminals through synaptic links. This causes painful sensation in nerve terminals, located in pulp-dentine junction [52]. However, there have been no conclusive evidence found; a number of studies showed that odontoblasts are matrix-forming cells, which are able to stimulate nerve terminals due to the absence of corresponding synapses[43].

The nerve theory, or direct innervation theory proves the presence of nerve terminals in dentine tubules, through which nerve stimuli are directly transferred. Nociceptive neurons innervating dental pulp must have receptors, which converse a specific stimulus into electric impulses [10].

Conventional therapy of dentine hypersensitivity is based on the application of desensitizing agents for local administration, which can be used both by professional and be prescribed for home use. A perfect desensitizing agent must not irritate or endanger pulp; must be relatively painless when applied or soon after application, be easily delivered, quick-relief, constantly effective and finally, it must not discolor dental structure [25]. Currently there are many desensitizing agents; however, there are no proved, safe and certified treatment regimens, which are superiorly effective as compared to others [14]. The treatment goals of both professional and home use implicate either interruption of neural response of the pulp or locking the sensitivity mechanism by tubules occlusion. Interruption of neural response can be reached via endodontia or exodontia on the one hand and dentine application of potassium salts on the other. In the latter case, treatment is usually performed with mouthwashes or toothpastes for home use. It has been considered that potassium salts decrease pulp nerve excitability and cause dentine desensitization [35, 41], but no clinical trial with reasonable design show no evidence of potassium being effective for teeth desensitization [45]. It is possible that potassium salts decreased dentine sensitivity due to placebo effect [41].

Professional treatment modes used for tubules occlusion normally include resins, varnishes, dentine binders or restorative materials [54].

Active compounds contained in desensitizing products can either block dentine tubule orifices thus isolating the orifice content, or directly decrease pulp nerve sensitivity. Strontium salts [40], fluorides [45], oxalates [17] and arginine/calcium [13, 44] precipitate and occlude tubules by forming a physical barrier. Thus, dentine liquid flow is blocked, and also direct stimulation of odontoblastic processes in dentine tubules by external irritants is prevented [56]. A suggested mechanism of glutaric aldehyde (another agent used to treat DHS) include reaction with serum albumin in dentine tubule liquid, which leads to formation of precipitate in tubules and further arctation or occlusion of tubules [1].

Conventional desensitizing agents often show unsatisfactory results, as their action is based on hydrodynamic theory and is focused on occlusion of open dentine tubules. It has been shown, however, that hypersensitivity persists even when dentine tubules are intentionally occluded with gutta-percha, and liquid movement becomes impossible [32]. Moreover, adhesive treatment modes for dentine hypersensitivity degrade with time and expose tubules again; relief is achieved only in the period from 3 to 6 months, after which patients start suffering from pain [2]. The clinical fact that dentine sensitivity symptom persists or aggravates, when dentine tubules are obliterated due to dental caries progression, ascertain the necessity to search for new treatment solution, which are not based on dental tubule isolation.

Recent research has shown that toothache occurs in the result of a complex mechanism explained by neurochemical interactions and anatomic structures. None of the known and previously used theories is self-sustaining and can give a complete explanation of processes within hypersensitivity. Moreover, the theories are not mutually exclusive, and the contribution of the mechanisms being in their bases, into the general condition of dentine sensitivity can vary.

However, most of the existing treatment methods are based on hydrodynamic theory, which makes them insufficient.

One group of biomolecules, which play the key role in the interpretation of various stimuli, represents a class of integral membrane proteins, named transient receptor potential (TRP) channels. TRP channels present a class of cation channels, which act as signal transducers, changing membrane potential or intracellular calcium concentration (Ca 2+). Expression of several members of this class was detected in afferent tooth nerves and odontoblasts [11]. One of the most common toothache triggers are thermal modifications in the oral cavity. It is considered that TRP play a leading role in physiology and act as transducers of thermal, mechanical and chemical stimuli [28]. According to sequence homology, a superfamily of mammal TRP channels is divided into six subfamilies: TRPC (canonic), TRPV (vanilloid), TRPM (melastatine), TRPA (ankyrin), TRPML (mucolipin) and TRPP (polycystic); as it is their expression which was shown in dental afferent neurons [7, 21, 29, 42].

Vanilloid channels with transient receptor potential (TRPV) were named based on activation of the founder member of this group with capsaicin, a vanilloid-like molecule; vanilloid transient receptor potential 1 (TRPV1) is of special interest for the authors. It was shown that the exposure to capsaicin and high temperatures leads to overexpression of TRPV1 and TRPV2, which contributes to hyperexcitability to thermal irritants in patients with pulpitis [12]. Increased TRPV1 expression was also shown when teeth were exposed to cold stimuli [42]. Both the function and the expression of TRPV1 are increased in case of inflammation and neuropathic pain. It was detected that various mediators produced during inflammation such as ATP, bradykinin and NGF, increase sensitivity to temperatures and protons, as well as enhance the activity of TRPV1 channel [9, 46].

Thus, TRPV1 is a nonselective cation channel, which is activated with various stimuli, including capsaicin, hazardous temperatures (nearly 42 °C), extra-cellular acid pH and bioactive lipids, such as lysophosphatidic acid [6, 30]. This makes it a possible therapeutic target when treating dentine hypersensitivity.

Several low molecule antagonists of TRPV1 channels have been tested in clinical trials in patients with toothache; but however, the results were poor, or clinical trials were early terminated [20, 36, 48] due to adverse effects. In one clinical trial, a pain-relieving effect of first generation TRPV1 antagonist, AMG 517 (Amgen), was tested after third molar extraction. However, the drug increased body temperature, which resulted in the termination of the trial [20]. Another TRPV1 antagonist (AZD1386, Astra-Zeneca) was tested in patients with sharp pain after lower third molar extraction, but showed only a short-term pain-relieving effect [48].

The second important subfamily is TRPC subfamily, or canonic TRP channels; it is the first subfamily of the studied mammal TRP channels.

TRPC channels, produced by homo- or heteromerous TRPC proteins, are Ca2+ penetrable nonselective cation channels.

Animal model studies showed that TRPC5 plays its role in dental sensitivity to cold [22]. TRPC5 blockers, such as HC-070 or ML204, effectively eliminated any response to cold and prevented pain, while ruizole, an agonist of this channel, increased jaw sensitivity. TRPC5 was detected in dental sensory neurons, as well as nearly in all predental odontoblasts in the dental pulp adjacent to the root. Outgrowths of TRPC5-positive odontoblasts in dentine tubules contacted sensory axons in the pulp-dentine junction and increased in close association in dentine tubules. Moreover, in the presence of dental pulp inflammation or caries development, expression of TRPC5 sensory nerve is significantly increased and extends to the degenerating dentine and the whole dental root, thus increasing sensitivity and painful sensation [3]. Beside the fact that TRPC5 is a cold sensor in odontoblasts, it can also indicate long-standing pain and be sensitive to oxidizing stress during inflammation, which makes it an effective target when treating dentine hypersensitivity [55].

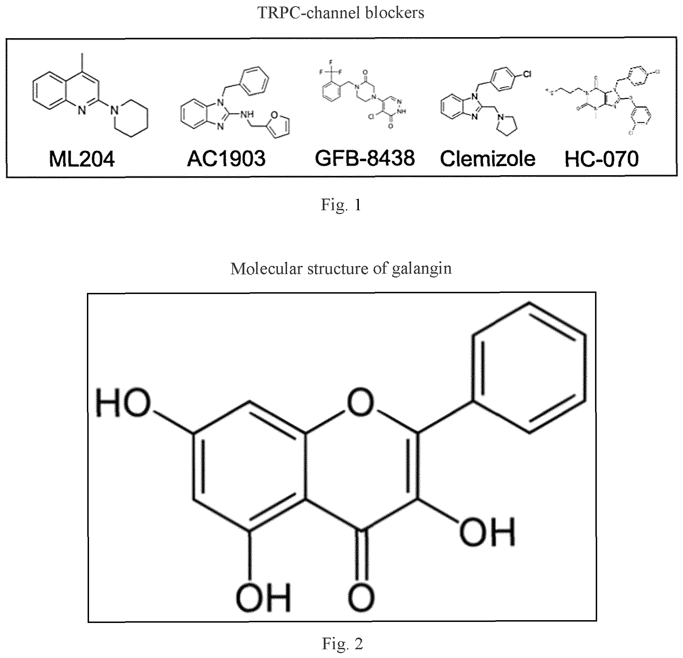

TRPC channel blockers (Fig. 1) exist, being well-known substances, but these molecules are of pharmaceutical grade, which impose certain restrictions to use it in cosmetic oral care products. These substances are expensive to use in commercial grade and are commercially unavailable. Thus, the problem for the authors of the present document is not only to find receptor inhibitors, but to find substances suitable in the sphere of oral care products and available both for the manufacturer and for the consumer.

Contingent on above remark, qualitative decrease of dentine hypersensitivity is a priority research area in the companies, developing oral care products. The available information on toothache distribution is not enough to clearly represent the mechanism of toothache distribution. However, it is evident at this point already that it is not enough to occlude dentine tubules or impact one of TRP responsible for sensitivity to stop the occurrence of sensitivity; it is necessary to develop a system, based on the complex approach of the impact on various receptors. The problem was to determine the components affecting TRPV1 and TRPC5 receptors, as they are responsible for toothache perception, resulting from most thermal and mechanical impact. The authors suggest that inhibition of these receptors can result in significant decrease of dentine sensitivity.

Such solution may be a combination, offered by the authors and comprising galangin flavonoid, which is able to impact the condition of TRPC5 channels, oleic acid, inhibiting TRPV1, and adenosine nucleoside.

Galangin is a natural flavonol of the flavonoids, which is contained in such plants as great galangal and Helichrysum aureonitens, Alpinia galanga rootstock, Mexican oregano and propolis. It is a biologically active component, to which many various properties are attributed in the literature, namely anti-inflammatory, antioxidative, anti carcinogenic and antiviral properties.

In the present research, the authors use artificial synthetic galangin molecule for pharmaceutical application (Fig. 2).

It had been shown before that galangin has an extremely low bioavailability with oral administration due to intensive metabolism, which casts some doubt on its property to have therapeutic effect [8]. Galangin is prone to rapid metabolic oxidation with various proteins, such as CYP1A1, which results in transformation into kaempferol [27]. In its turn, kaempferol either does not affect TRPC5 receptors, or shows a significantly milder impact than that of galangin [39]. It was shown that pure galangin is practically insoluble in water [34]. All the abovementioned made the use of galangin in oral care products impractical.

Oleic acid (OA) is widely spread in plant, animal, nut, sea and algal lipids. OA is a 18-carbon fatty acid, containing cis-double bond in 9-carbonic position. In the modem fatty acid nomenclature, double bonds are counted from the terminal, not carboxy terminus, and OA is normally denoted as omega-9 fatty acid. Oleic acid contributes to body metabolism and participates in membrane formation. A number of studies proves its immunomodulating, vulnerary and anti-inflammatory properties [49].

Thanks to its unique architecture and composition, external skin layer is a barrier for small molecule penetration, which thus protects lower skin layers. Oleic acid is able to decrease barrier properties of stratum corneum, selectively affecting extra cellular lipids, which represent the main regulatory channel for small molecule penetration [37]. Such booster molecules are able to modify biochemical environment of the skin as well as to disrupt ordered arrangement of intercellular lipid domain [53]. Moreover, it was shown that oleic acid is able to modify epidermic cell morphology.

Langerhans cells are stratum corneum cells, participating in immune response and having regulator impact on keratinocyte proliferation and differentiation. The shortage of or damage to these cells in the oral cavity can result in immunological suppression which contributes to declined immune response, occurrence of inflammation and irritation [5]. It was shown that the application of oleic acid on the skin results in pore formation on the skin surface, which in its turn can cause irritation. Enlarged pores can also contribute to improved access of various molecules to soft tissues and dentine, thus increasing dental sensitivity [51].

Adenosine is a purine nucleoside base, often known for its adenosine triphosphate, or ATP, molecule; it is widely used in the body and in the basic metabolism [50]. Adenosine is a modulator having comprehensive and generally inhibiting effect on neuron activity [16]. It was shown that adenosine receptor (AR) activation can impact nociceptive, inflammatory and neuropathic pain conditions [26]. Agonists of adenosine receptor showed their antinociceptive (pain-relieving) effects in case of TRPV1 activation. Moreover, TRPV1 stimulation with capsaicin causes excitotoxicity, which finally results in breaking primary afferent nerve fibers [44]. As TRPV1-mediated cell death is connected with peripheric neuropathy, it was determined that adenosine is able to inhibit capsaicin-mediated apoptosis [33]. In the aggregate, this data allow suggesting that adenosine is able to serve as endogenic inhibitor of TRPV1 activity, directly interacting with receptor protein [47].

Scientists demonstrated that oleic acid consumption decreases availability and transport rate of adenosine [4]. Thus, the co-use of these components can lead to the decreased effect of adenosine, as well as to the slower delivery of its molecules to receptors.

The authors of this invention unexpectedly found out that the combination of oleic acid with galangin and adenosine prevents the presentation of negative impact of these components and contributes to galangin availability, which is proved by therapeutic effect. This product does not cause irritation and discomfort when used, but significantly decreases dentine and periodontal tissue sensitivity in consumers.

**Table 1. Basic formulation of toothpaste to include the system:**

| Ingredient | % |
|---|---|
| Purified water | Max. 99% |
| Surfactant (Sodium Lauroyl Sarcosinate) | 0,1%-10% |
| Abrasive (Hydrated Silica) | 0,5%-30% |
| Solubilizer (Glycerin) | 0,1%-10% |
| Preservative (Potassium Sorbate and Sodium Benzoate of Benzyl Alcohol) | 0,01%-3% |

### Studies

The purpose of the suggested randomized controlled study is an efficacy estimation of various protocols to manage DHS in patients with hypersensitivity. This protocol complies with the recommendations of the Standard Randomized Study Protocol, presented in Table 2.

**Table 2. Schedule of enrollment, intervention and assessment of the study**

| | | | | | Close-out |
|---|---|---|---|---|---|
| | | Enrolment | Allocation | 0 | t1 |
| Enrolment: | | | | | |
| | Eligibility screen | x | | | |
| | Allocation | | x | | |

| Interventions: | | | | | |
|---|---|---|---|---|---|
| | Positive control | | | x | x |
| | Negative control | | | x | x |
| | Galangin | | | x | x |
| | Adenosine | | | x | x |
| | Oleic acid | | | x | x |
| | Galangin + Adenosine + Oleic acid | | | x | x |

| Assessments: | | | | | |
|---|---|---|---|---|---|
| | Cold pain | | | x | x |
| | Hot pain | | | x | x |

| | | | | | |
|---|---|---|---|---|---|
| 0, baseline; t1, immediately after treatment; | | | | | |

### Eligibility criteria

- Age from 18 to 35 years old
- Good overall health;
- At least one DHS tooth with sensitivity over or equal to 4 (VAS). Withdrawal criteria
- Active caries or defective restorations on the tested tooth;
- Considerable loss of dentine, requiring remedial treatment or paradental surgery;
- Presence of veneers;
- Any professional desensitizing treatment during previous 6 months;
- Use of desensitizing toothpaste during previous 3 months.
- Use of anti-inflammatory medicine or painkillers during enrollment;
- Currently pregnant of breast-feeding.

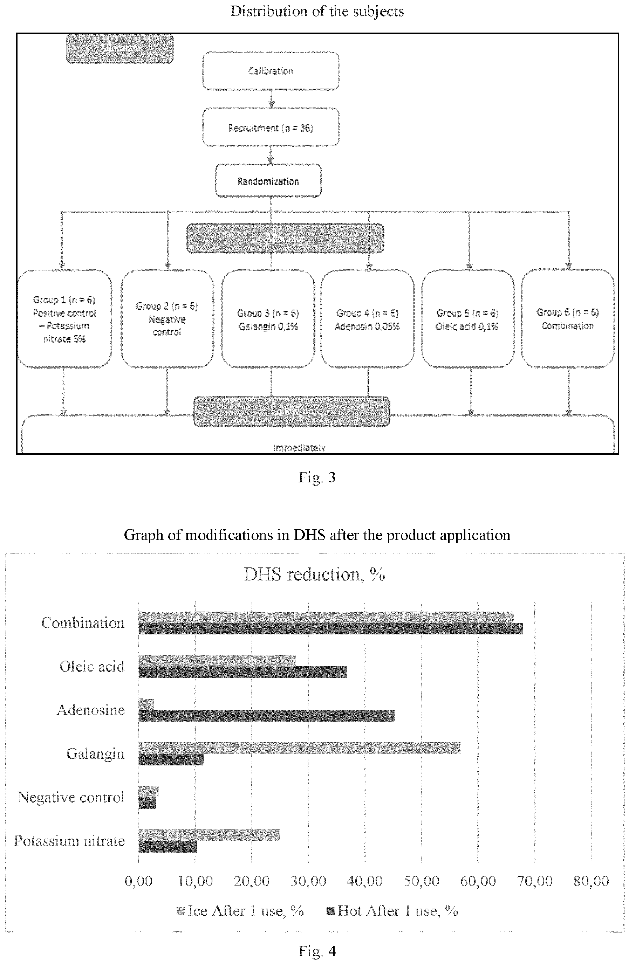

The subjects were randomized into 6 treatment groups, two control groups of which were negative and positive controls (Fig. 3).

### Flowchart of the study

For random distribution of subjects, randomization was made as a draw with the website www.randomizer.org. An average value of initial points according to DHS degree was determined for each subject, and the subjects were randomized into treatment groups providing the equivalent initial DHS points for various treatment modes. All the hypersensitive teeth in each subject were estimated with the use of DHS; an average value was considered for the baseline. All the hypersensitive teeth were subjected and re-estimated. Next, and average value was calculated for the final estimation of sensitivity.

Two weeks before the study the subjects had a washout period, during which they used only those oral care products, which were provided by the investigators. These products were used by them till the end of the study. An oral care set included soft toothbrush and toothpaste for general oral care.

All the subjects and sensitivity degree assessor were blinded in the study; they did not know which group a subject belongs to.

The treatment groups received mouthwashes with the following systems;

**Table 3. Basic formulation of mouthwash to include the system:**

| Ingredient | % |
|---|---|
| Purified water | Max. 99% |
| Surfactant | 0,1%-10% |
| Solubilizer | 0,1%-10% |
| Preservative | 0,01%-3% |

**Table 4. Basic formulation of toothpaste to include the system:**

| Ingredient | % |
|---|---|
| Purified water | Max. 99% |
| Surfactant | 0,1%-10% |
| Abrasive | 0,5%-30% |
| Solubilizer | 0,1%-10% |
| Preservative | 0,01%-3% |

| | |
|---|---|
| - Group 1 - Mouthwash with Potassium nitrate 5% (Positive control) Clear uncolored solution. - Group 2 - Mouthwash without active ingredient (Negative control) Clear uncolored solution. - Group 3 - Mouthwash with Galangin 0,1% Solution of light-yellow color. - Group 4 - Mouthwash with Adenosine 0,05% Clear uncolored solution. - Group 5 - Mouthwash with Oleic acid 0, 1% Solution of light-yellow color. - Group 6 - Mouthwash with Galangin, Adenosine and Oleic acid Solution of light-yellow color. | |

### Results of the study.

The primary result expected in the study was the decrease of dental hypersensitivity after the first application of the product. The result of the system application was calculated with a visual analogue scale, which registers sensitivity with a 10 cm line. The line extremums are the pain limits, which the subject can feel from external irritant (0 - no pain; 10 - severe pain). First, dental sensitivity was registered with an ice cube and an applicator heated to 90C. After the first application, sensitivity to external irritant was registered with an ice cube and an applicator heated to 90C.

The tested sample size was 36 subjects. The data was collected and then filtered out from errors and processed with the standard functionality of MS Excel. The charts were made with Microsoft Excel software.

**Table 5. Results of hypersensitivity assessment study**

| **Group** | **1** | **Mean** | **2** | **Mean** | **3** | **Mean** | **4** | **Mean** | **5** | **Mean** | **6** | **Mean** |
|---|---|---|---|---|---|---|---|---|---|---|---|---|
| Before Hot | | | | | | | | | | | | |
| 1 | 5 | 6,7 | 7 | 6,5 | 3 | 5,2 | 8 | 7,3 | 5 | 6,8 | 7 | 7,8 |
| 2 | 6 | | 6 | | 6 | | 9 | | 9 | | 8 | |
| 3 | 5 | | 8 | | 4 | | 7 | | 6 | | 7 | |
| 4 | 8 | | 7 | | 7 | | 6 | | 6 | | 8 | |
| 5 | 8 | | 4 | | 5 | | 5 | | 8 | | 9 | |
| 6 | 8 | | 7 | | 6 | | 9 | | 7 | | 8 | |

| Before Ice | | | | | | | | | | | | |
|---|---|---|---|---|---|---|---|---|---|---|---|---|
| 1 | 8 | 7,6 | 9 | 8,3 | 5 | 5,8 | 7 | 7 | 6 | 7,2 | 7 | 8 |
| 2 | 7 | | 8 | | 6 | | 8 | | 9 | | 9 | |
| 3 | 8 | | 9 | | 5 | | 7 | | 8 | | 8 | |
| 4 | 8 | | 9 | | 6 | | 6 | | 5 | | 7 | |
| 5 | 6 | | 7 | | 6 | | 8 | | 8 | | 9 | |
| 6 | 9 | | 8 | | 7 | | 6 | | 7 | | 8 | |

| Hot After 1 use | | | | | | | | | | | | |
|---|---|---|---|---|---|---|---|---|---|---|---|---|
| 1 | 5 | 6 | 7 | 6,3 | 4 | 4,6 | 5 | 4 | 3 | 4,3 | 3 | 2,5 |
| 2 | 5 | | 6 | | 6 | | 6 | | 5 | | 3 | |
| 3 | 4 | | 7 | | 3 | | 4 | | 4 | | 2 | |
| 4 | 7 | | 5 | | 5 | | 2 | | 6 | | 2 | |
| 5 | 8 | | 6 | | 5 | | 4 | | 4 | | 3 | |
| 6 | 7 | | 7 | | 5 | | 3 | | 4 | | 2 | |

| Ice After 1 use | | | | | | | | | | | | |
|---|---|---|---|---|---|---|---|---|---|---|---|---|
| 1 | 5 | 5,7 | 8 | 8 | 3 | 2,5 | 8 | 6,8 | 8 | 5,2 | 3 | 2,7 |
| 2 | 4 | | 8 | | 2 | | 9 | | 4 | | 3 | |
| 3 | 5 | | 7 | | 4 | | 6 | | 6 | | 3 | |
| 4 | 7 | | 9 | | 1 | | 6 | | 3 | | 2 | |
| 5 | 7 | | 9 | | 3 | | 5 | | 6 | | 3 | |
| 6 | 6 | | 7 | | 2 | | 7 | | 4 | | 2 | |

**Table 6. Modifications in HS after the product application**

| **Group** | **Potassium nitrate** | **Negative control** | **Galangin** | **Adenosine** | **Oleic acid** | **Combination** |
|---|---|---|---|---|---|---|
| Hot After 1 use, % | 10.4 | 3,1 | 11.5 | 45.2 | 36.8 | 67.9 |
| Ice After 1 use, % | 25 | 3.6 | 56,9 | 2.8 | 27.8 | 66.3 |

The chart showing modifications in HS after the product application is presented in Figure 4.

In the result of the experiment the authors proved that the combined use of adenosine, galangin and oleic acid removes sensitivity after the first application by an average of 66%.

Also, in the experiment, the authors unexpectedly found out that the system with adenosine, galangin and oleic acid (a combined system) removes sensitivity not only in teeth, but also removes painful sensation in periodontal tissue, thus removing painful sensation for the period from 4 to 9 hours depending on the respondent.

### REFERENCES

1. Arrais C. A. G., Chan D. C. N., Giannini M. Effects of desensitizing agents on dentinal tubule occlusion // Journal of Applied Oral Science. 2004. N° 2 (12).
2. Bartold P. M. Dentinal hypersensitivity: A review // Australian Dental Journal. 2006. T. 51.N° 3.
3. Bernal L. [et al.]. Odontoblast TRPC5 channels signal cold pain in teeth // Science Advances. 2021. N° 13 (7).
4. Blackmore V. L., Meckling-Gill K. A. Fish oil and oleic acid-rich oil feeding alter nucleoside uptake in human erythrocytes // The Journal of Nutritional Biochemistry. 1995. N° 8 (6).
5. Boelsma E. [et al.]. Assessment of the potential irritancy of oleic acid on human skin: Evaluation in vitro and in vivo // Toxicology in Vitro. 1996. N° 6 (10).
6. Caterina M. J. [et al.]. The capsaicin receptor: A heat-activated ion channel in the pain pathway // Nature. 1997. N° 6653 (389).
7. Chaudhary P., Martenson M. E., Baumann T. K. Vanilloid receptor expression and capsaicin excitation of rat dental primary afferent neurons // Journal of Dental Research. 2001. N° 6 (80).
8. Chen F. [et al.]. Differential systemic exposure to galangin after oral and intravenous administration to rats // Chemistry Central Journal. 2015. N° 1 (9).
9. Chuang H. H. [et al.]. Bradykinin and nerve growth factor release the capsaicin receptor from PtdIns(4,5)P2-mediated inhibition // Nature. 2001. N° 6840 (411).
10. Chung G., Jung S. J., Oh S. B. Cellular and molecular mechanisms of dental nociception // Journal of Dental Research. 2013. T. 92. N° 11.
11. Chung G., Oh S. B. TRP Channels in Dental Pain // The Open Pain Journal. 2013. N° 1 (6).
12. Chung M. K. [et al.]. Lipopolysaccharide-induced pulpitis up-regulates TRPV1 in trigeminal ganglia // Journal of Dental Research. 2011. N° 9 (90).
13. Cummins D. Recent advances in dentin hypersensitivity: Clinically proven treatments for instant and lasting sensitivity relief // American Journal of Dentistry. 2010. T. 23. N° SPECIAL ISSUE A.
14. Dababneh R., Khouri A., Addy M. Dentine hypersensitivity - an enigma? a review of terminology, mechanisms, aetiology and management // British Dental Journal. 1999. N° 11 (187).
15. Douglas-de-Oliveira D. W. [et al.]. Effect of dentin hypersensitivity treatment on oral health related quality of life - A systematic review and meta-analysis // Journal of Dentistry. 2018. T. 71.
16. Dunwiddie T. V., Masino S. A. The role and regulation of adenosine in the central nervous system // Annual Review of Neuroscience. 2001. T. 24.
17. Ehrlich J. [et al.]. Residual Fluoride Concentrations and Scanning Electron Microscopic Examination of Root Surfaces of Human Teeth After Topical Application of Fluoride In Vivo // Journal of Dental Research. 1975. N° 4 (54).
18. Fischer C., Fischer R. G., Wennberg A. Prevalence and distribution of cervical dentine hypersensitivity in a population in Rio de Janeiro, Brazil // Journal of Dentistry. 1992. N° 5 (20).
19. Flynn J., Galloway R., Orchardson R. The incidence of «hypersensitive» teeth in the West of Scotland // Journal of Dentistry. 1985. N° 3 (13).
20. Gavva N. R. [et al.]. Pharmacological blockade of the vanilloid receptor TRPV1 elicits marked hyperthermia in humans // Pain. 2008. N° 1-2 (136).
21. Gibbs J. L., Melnyk J. L., Basbaum A. I. Differential TRPV1 and TRPV2 channel expression in dental pulp // Journal of Dental Research. 2011. N° 6 (90).
22. Gibbs J. L., Urban R., Basbaum A. I. Paradoxical surrogate markers of dental injury-induced pain in the mouse // Pain. 2013. N° 8 (154).
23. Goh V., Corbet E. F., Leung W. K. Impact of dentine hypersensitivity on oral health-related quality of life in individuals receiving supportive periodontal care // Journal of Clinical Periodontology. 2016. N° 7 (43).
24. Graf H., Galasse R. Morbidity, prevalence and intra-oral distribution of hypersensitive teeth // Journal of dental research. 1977. N° Special Issue A (56).
25. Grossman L. I. A Systematic Method for the Treatment of Hypersensitive Dentin // The Journal of the American Dental Association (1922). 1935. N° 4 (22). C. 592-602.
26. Haddad M. [et al.]. The role of adenosine receptor ligands on inflammatory pain: possible modulation of TRPV1 receptor function // Inflammopharmacology. 2023. N° 1 (31).
27. Hamada M. [et al.]. Metabolites of galangin by 2,3,7,8-tetrachlorodibenzo-pdioxin-inducible cytochrome P450 1A1 in human intestinal epithelial Caco-2 cells and their antagonistic activity toward aryl hydrocarbon receptor // Journal of Agricultural and Food Chemistry. 2010. N° 13 (58).
28. Karim I. A. El [et al.]. Human odontoblasts express functional thermo-sensitive TRP channels: Implications for dentin sensitivity // Pain. 2011. N° 10 (152).
29. Kim H. Y. [et al.]. Characterization of dental nociceptive neurons // Journal of Dental Research. 2011. N° 6 (90).
30. Liao M. [et al.]. Structure of the TRPV1 ion channel determined by electron cryo-microscopy // Nature. 2013. N° 7478 (504).
31. Lima T. C. [et al.]. Oral Health-Related Quality of Life Before and After Treatment of Dentin Hypersensitivity With Cyanoacrylate and Laser // Journal of Periodontology. 2017. N° 2 (88).
32. Linsuwanont P. [et al.]. Thermal stimulation causes tooth deformation: A possible alternative to the hydrodynamic theory? // Archives of Oral Biology. 2008. N° 3 (53).
33. Maccarrone M. [et al.]. Anandamide induces apoptosis in human cells via vanilloid receptors. Evidence for a protective role of cannabinoid receptors // Journal of Biological Chemistry. 2000. N° 41 (275).
34. Mak K. K. [et al.]. Galangin's potential as a functional food ingredient // Journal of Functional Foods. 2018. T. 46.
35. Markowitz K., Bilotto G., Kim S. Decreasing intradental nerve activity in the cat with potassium and divalent cations // Archives of Oral Biology. 1991. N° 1 (36).
36. Mickle A. D., Shepherd A. J., Mohapatra D. P. Nociceptive TRP channels: Sensory detectors and transducers in multiple pain pathologies // Pharmaceuticals. 2016. T. 9. N° 4.
37. Naik A. [et al.]. Mechanism of oleic acid-induced skin penetration enhancement in vivo in humans // Journal of Controlled Release. 1995. N° 3 (37).
38. Närhi M. V. O., Hirvonen T. The response of dog intradental nerves to hypertonic solutions of CaCl2 and NaCl, and other stimuli, applied to exposed dentine // Archives of Oral Biology. 1987. N° 11 (32).
39. Naylor J. [et al.]. Natural and synthetic flavonoid modulation of TRPC5 channels // British Journal of Pharmacology. 2016. N° 3 (173).
40. Olley R. C. [et al.]. An in situ study investigating dentine tubule occlusion of dentifrices following acid challenge // Journal of Dentistry. 2012. N° 7 (40).
41. Orchardson R., Gillam D. G. The Efficacy of Potassium Salts as Agents for Treating Dentin Hypersensitivity // Journal of Orofacial Pain. 2000. N° 1 (14).
42. Park C. K. [et al.]. Functional expression of thermo-transient receptor potential channels in dental primary afferent neurons: Implication for tooth pain // Journal of Biological Chemistry. 2006. N° 25 (281).
43. Pashley D. H. Dynamics of the Pulpo-Dentin Complex // Critical Reviews in Oral Biology & Medicine. 1996. N° 2 (7). C. 104-133.
44. Petrou I. [et al.]. A breakthrough therapy for dentin hypersensitivity: How dental products containing 8% arginine and calcium carbonate work to deliver effective relief of sensitive teeth // Journal of Clinical Dentistry. 2009. N° 1 (20).
45. Poulsen S. [et al.]. Potassium containing toothpastes for dentine hypersensitivity // Cochrane Database of Systematic Reviews. 2006.
46. Premkumar L. S., Agarwal S., Steffen D. Single-channel properties of native and cloned rat vanilloid receptors // Journal of Physiology. 2002. T. 545. N° 1.
47. Puntambekar P. [et al.]. Direct Interaction of Adenosine with the TRPV1 Channel Protein // Journal of Neuroscience. 2004. N° 14 (24).
48. Quiding H. [et al.]. TRPV1 antagonistic analgesic effect: A randomized study of AZD1386 in pain after third molar extraction // Pain. 2013. N° 6 (154).
49. Sales-Campos H. [et al.]. An Overview of the Modulatory Effects of Oleic Acid in Health and Disease // Mini-Reviews in Medicinal Chemistry. 2013. N° 2 (13).
50. Samsel M., Dzierzbicka K., Trzonkowski P. Adenosine, its analogues and conjugates // Post 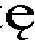 py Higieny i Medycyny Doświadczalnej. 2013. (67).
51. Stillman M. A., Maibach H. I., Shallita A. R. Relative irritancy of free fatty acids of different chain length // Contact Dermatitis. 1975. N° 2 (1).
52. Sufyan Garoushi N. B. Dentine Hypersensitivity: A Review // Dentistry. 2015. N° 09 (05).
53. Touitou E. [et al.]. Oleic acid, a skin penetration enhancer, affects Langerhans cells and corneocytes // Journal of Controlled Release. 2002. N° 1-3 (80).
54. Trowbridge H. O., Silver D. R. A review of current approaches to in-office management of tooth hypersensitivity. // Dental clinics of North America. 1990. T. 34. N° 3.
55. Yamamoto S., Takahashi N., Mori Y. Chemical physiology of oxidative stress-activated TRPM2 and TRPC5 channels // Progress in Biophysics and Molecular Biology. 2010. T. 103.N° 1.
56. Zhao X. [et al.]. Protective effects of resin sealant and flowable composite coatings against erosive and abrasive wear of dental hard tissues // Journal of Dentistry. 2016. (49).

## Claims

1. A composition comprising
(A) galangin,
(B) oleic acid, and
(C) adenosine.

2. The composition of claim 1, wherein said composition comprises the galangin as per the following wt.% sub-ranges in said composition: 0.01 - 20.00, 0.02 - 15.00, 0.03 - 10.00, 0.04 - 5.00, 0.05 - 4.00, 0.06 - 3.00, 0.07 - 2.00, 0.08 - 1.50, 0.09 - 1.10, 0.01 - 2.00, 0.02 - 1.50, 0.03 - 1.00, 0.04 - 0.50, 0.05 - 0.40, 0.06 - 0.30, 0.07 - 0.20, 0.08 - 0.15, 0.09 - 0.11, and combinations thereof.

3. The composition of any of the preceding claims, wherein said composition comprises said oleic acid as per the following wt.% ranges in said composition: 0.01 - 20.00, 0.02 - 15.00, 0.03 - 10.00, 0.04 - 5.00, 0.05 - 4.00, 0.06 - 3.00, 0.07 - 2.00, 0.08 - 1.50, 0.09 - 1.10, 0.01 - 2.00, 0.02 - 1.50, 0.03 - 1.00, 0.04 - 0.50, 0.05 - 0.40, 0.06 - 0.30, 0.07 - 0.20, 0.08 - 0.15, 0.09 - 0.11, and combinations thereof.

4. The composition of any of the preceding claims, wherein said composition comprises said adenosine as per the following wt.% ranges in said composition: 0.005 - 10.000, 0.010 - 7.500, 0.015 - 5.000, 0.020 - 2.500, 0.025 - 2.000, 0.030 - 1.500, 0.035 - 1.000, 0.040 - 0.750, 0.045 - 0.550, 0.005 - 1.000, 0.010 - 0.750, 0.015 - 0.500, 0.020 - 0.250, 0.025 - 0.200, 0.030 - 0.150, 0.035 - 0.100, 0.040 - 0.075, 0.045 - 0.055, and combinations thereof.

5. The composition of claim 1, wherein said composition comprises the following wt.% options in said composition:
| Option | galangin | oleic acid | adenosine |
|---|---|---|---|
| 1 | 0.010 - 20.000 | 0.010 - 20.000 | 0.005 - 10.000 |
| 2 | 0.020 - 15.000 | 0.020 - 15.000 | 0.010 - 7.500 |
| 3 | 0.030 - 10.000 | 0.030 - 10.000 | 0.015 - 5.000 |
| 4 | 0.040 - 5.000 | 0.040 - 5.000 | 0.020 - 2.500 |
| 5 | 0.050 - 4.000 | 0.050 - 4.000 | 0.025 - 2.000 |
| 6 | 0.060 - 3.000 | 0.060 - 3.000 | 0.030 - 1.500 |
| 7 | 0.070 - 2.000 | 0.070 - 2.000 | 0.035 - 1.000 |
| 8 | 0.080 - 1.500 | 0.080 - 1.500 | 0.040 - 0.750 |
| 9 | 0.090 - 1.100 | 0.090 - 1.100 | 0.045 - 0.550 |
| 10 | 0.010 - 2.000 | 0.010 - 2.000 | 0.005 - 1.000 |
| 11 | 0.020 - 1.500 | 0.020 - 1.500 | 0.010 - 0.750 |
| 12 | 0.030 - 1.000 | 0.030 - 1.000 | 0.015 - 0.500 |
| 13 | 0.040 - 0.500 | 0.040 - 0.500 | 0.020 - 0.250 |
| 14 | 0.050 - 0.400 | 0.050 - 0.400 | 0.025 - 0.200 |
| 15 | 0.060 - 0.300 | 0.060 - 0.300 | 0.030 - 0.150 |
| 16 | 0.070 - 0.200 | 0.070 - 0.200 | 0.035 - 0.100 |
| 17 | 0.080 - 0.150 | 0.080 - 0.150 | 0.040 - 0.075 |
| 18 | 0.090 - 0.110 | 0.090 - 0.110 | 0.045 - 0.055 |
| 19 | 0.100 | 0.100 | 0.050 |

6. The composition of any of the preceding claims, wherein said composition is assembled as
i) a single composition comprising components (A), (B) and (B), or
ii) a kit-of-parts, wherein said kit-of-parts separates components (A), (B) and (C) in any combination, including
- (A) combined with (B) and separated from (C),
- (A) combined with (C) and separated from (B),
- (B) combined with (C) and separated from (A), and
- (A), (B) and (C) separated from each other.

7. The composition of any of the preceding claims, wherein said
- galangin has the CAS number 548-83-4;
- oleic acid has the CAS number CAS 112-80-1; and
- adenosine has the CAS number 58-61-7.

8. The composition of any of the preceding claims, wherein said composition has a pH of 4.0 to 8.5, preferably selected from the following pH sub-ranges: 4.5-8.0, 5.0-7.5, 5.5-7.0, 6.0-6.5, and combinations thereof.

9. The composition of any of the preceding claims, wherein said composition is an oral care composition, wherein said oral care composition is preferably selected from the group consisting of: a mouthwash, a toothpaste, an oral foam, an oral gel, and preferably a mouthwash.

10. The composition of any of the preceding claims, wherein said composition is a formulation selected from the following: film, aerosol, suspension, solution, tincture, cream, paste, lotion, ointment, gel, powder, granulate, wherein said formulation is preferably a solution.

11. Use of the composition of any of the preceding claims for non-medical and/or aesthetic use, wherein said use is teeth and/or periodontium treatment, including cleaning, brushing and massage.

12. Pharmaceutical preparation comprising the composition of any of claims 1-10.

13. The composition of any of claims 1-10 for use as a medicament.

14. The composition for use of any of claims 1-10 for use in the prevention from or treatment of a disease or symptoms of a disease in a subject, wherein said disease is dentin hypersensitivity (DHS).

15. The composition for use of any of claims 1-10 for use in the prevention from or treatment of a disease or symptoms of a disease in a subject, wherein said disease is periodontitis and/or gingivitis.
